Europäisches Patentamt

(19) European Patent Office

Office européen des brevets

(11) Numéro de publication: **0 061 390**

**A1**

(12) **DEMANDE DE BREVET EUROPEEN**

(21) Numéro de dépôt: **82400457.6**

(22) Date de dépôt: **12.03.82**

(51) Int. Cl.³: **A 61 B 6/00**
**A 61 B 6/02**

(30) Priorité: **24.03.81 FR 8105858**

(43) Date de publication de la demande:
**29.09.82 Bulletin 82/39**

(84) Etats contractants désignés:
**DE GB NL SE**

(71) Demandeur: **THOMSON-CSF**
**173, Boulevard Haussmann**
**F-75360 Paris Cedex 08(FR)**

(72) Inventeur: **Chambron, Edmond**
**THOMSON-CSF SCPI 173, bld Haussmann**
**F-75360 Paris Cedex 08(FR)**

(74) Mandataire: **Dubreuil, Annie et al,**
**THOMSON-CSF SCPI 173, Bld Haussmann**
**F-75360 Paris Cedex 08(FR)**

(54) **Statif radiologique intégré.**

(57) L'invention concerne un statif radiologique intégré comprenant essentiellement un tube à rayons X (3) et un sériographe (7) disposés sous un panneau porte-patient (6). Le tube à rayons X (3) coopère avec un amplificateur de luminance (20) extérieur au statif, et le sériographe (7) avec un tube à rayons X (26) également extérieur au statif; ces moyens sont agencés de telle sorte que le sériographe (7) peut se superposer au tube à rayons X (3), ce qui permet le passage rapide d'une phase de travail en radioscopie à une phase de travail en radiographie et réciproquement.

L'invention s'applique notamment aux examens radiologiques vasculaires.

FIG_2

EP 0 061 390 A1

1

## STATIF RADIOLOGIQUE INTEGRE

La présente invention concerne le domaine de la radiologie et notamment celui des radiodiagnostics.

Les examens radiologiques d'un patient peuvent nécessiter de nombreux moyens qui en fonctionnement sont corrélés. Il existe dans l'état actuel de la technique des systèmes en éléments séparés dont l'assemblage pour un travail en commun ne permet pas d'optimiser leur fonctionnement, et ne se prête pas à une bonne protection contre les radiations ionisantes.

Dans le cas plus particulier des examens vasculaires, l'exploration radiologique du patient peut exiger de très nombreuses successions de cycles constitués par l'examen radioscopique de la zone étudiée, suivi de sa ou ses radiographies ; une cadence rapide de ces suites d'opérations est souvent nécessaire à la réussite de l'examen.

Avec les équipements actuellement connus, l'exploration du patient des pieds à la tête demande des déplacements longitudinaux successifs du panneau porte-patient, ainsi que des réalignements du couple source de rayonnement X - amplificateur de luminance dus notamment aux alternances de radioscopie et de radiographie. Ces opérations étant effectuées manuellement, elles sont sources de fatigue pour le patient et pour le personnel utilisateur et génèrent des pertes de temps importantes.

On trouve dans l'art antérieur des appareils ou l'alignement de la source de rayonnement X et de l'amplificateur de luminance associé est conservé, ceux-ci étant respectivement monté à chaque extrémité d'un support courbe. La demande de brevet allemand publiée sous le N° 2342704, décrit un appareil, dans lequel la rotation de 180° du dispositif de soutient du support recourbé, à chaque extrémité duquel sont respectivement fixés la source de rayonnement X et l'amplificateur de luminance est possible. Un tel agencement peut permettre à la source de rayonnement X, d'être situé au-dessous du panneau porte-patient pour la phase radioscopie, ou au-dessus pour la phase de radiographie ; mais le mouvement qui est nécessaire peut être gêné par le panneau porte-patient, qui le cas échéant devra être déplacé longitudinalement, nuisant ainsi au déroulement rapide de l'examen.

D'autre part, le déplacement longitudinal du panneau porte-patient que l'on retrouve dans les autres équipements existant dans ce domaine implique un porte à faux important du plan de table, d'ou l'obligation de prévoir des rails de déplacement longitudinaux ayant un moment d'inertie important et dont la présence gêne l'exploration radiologique face/profil du patient.

La présente invention concerne un statif de radiologie dont l'agencement permet une intégration et une automatisation de ses éléments, de sorte qu'il ne présente pas les inconvénients des équipements connus.

Un tel agencement permet le passage rapide et précis d'une phase de travail en radioscopie a une phase de travail en radiographie et réciproquement, ceci à l'aide d'un matériel simple, compact et de manipulation aisée.

L'invention concerne plus particulièrement un statif de radiologie comportant notamment : un panneau porte-patient ; un premier moyen intérieur au statif situé sous ce panneau, ce premier moyen intérieur étant soit une source, soit un détecteur de rayonnement, lié à un premier moyen extérieur au statif situé au-dessus du panneau porte-patient et étant selon la nature du premier moyen intérieur soit un détecteur soit une source de rayonnement formant ainsi, dans chaque cas, un premier couple, source - détecteur, déplaçable longitudinalement tout le long du panneau porte-patient ; caractérisé en ce qu'il comporte en outre un second moyen intérieur au statif situé sous le panneau porte-patient et un second moyen extérieur au statif situé au-dessus du panneau porte-patient, ces deux derniers moyens constituant un second couple source - détecteur déplaçable tout le long du panneau porte-patient, les chemins de déplacement du premier et second moyen intérieur étant parallèles et agencés de telle sorte que les déplacements de ces deux moyens ne se gênent pas mutuellement et permettent la substitution d'un couple par l'autre.

D'autres caractéristiques de l'invention apparaîtront à la lecture de la description suivante donnée à titre d'exemple qui la fera mieux comprendre. Elle s'appuie pour plus de clarté sur des dessins annexés au nombre de 2.

- La figure 1 montre en perspective une vue du statif suivant l'invention pris isolément.

- La figure 2 représente en perspective les éléments essentiels du statif suivant l'invention, ainsi que des moyens extérieurs avec lequel ils coopèrent.

Pour plus de clarté les même éléments portent les mêmes références dans toutes les figures.

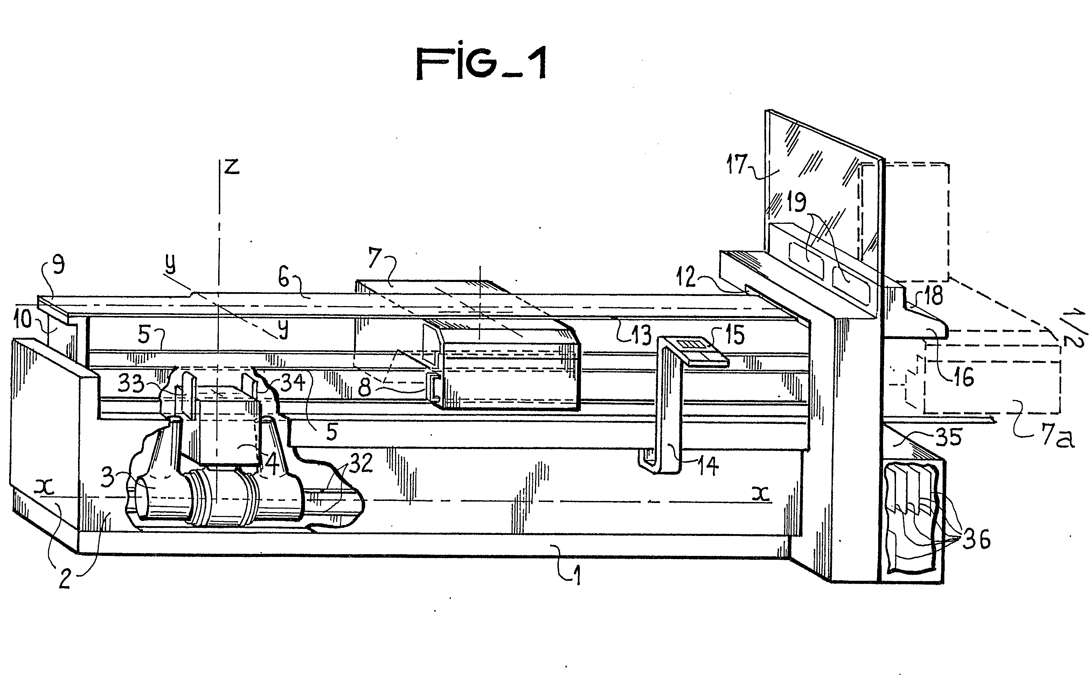

Comme le montre la figure 1, le bâti 1 repose au sol sans porte à faux ; il est garni sur son pourtour latéral d'un blindage 2, constitué par exemple de tôles d'acier intérieurement revêtues d'une pellicule de plomb, afin d'augmenter la protection contre les rayonnements ionisants.

A un niveau inférieur de bâti 1, existent : un chemin de déplacement longitudinal constitué par les rails 32 (partiellement visibles par l'ouverture pratiquée dans le dessin du blindage 2) ; un moyen intérieur 3 qui peut être indifféremment soit une source soit un détecteur de rayonnement, monté sur un support motorisé non représenté sur le dessin, circulant sur les rails 32, et rendant ainsi le moyen 3 capable d'un déplacement longitudinal selon l'axe x-x dans tout l'espace intérieur du bâti 1 ; dans l'exemple décrit, il s'agit d'une source de rayonnement X dont le tube à rayons X 3 (visible grâce à l'ouverture pratiquée dans le dessin du blindage 2) comporte un diaphragme plombé 4. L'axe du rayonnement est sur un axe vertical Z dit de travail qui passe entre les rails 5 pour traverser le panneau porte-patient 6 perpendiculairement à son plan et lorsque ce moyen intérieur se déplace, l'axe de travail se déplace le long de l'axe longitudinal du panneau porte-patient lui-même parallèle à l'axe x-x, et ce, en fonction des positions occupées par le tube à rayons X 3.

A un niveau supérieur au bâti 1 mais situé à l'intérieur de celui-ci, se trouve un deuxième moyen intérieur qui est un détecteur de rayonnement quelconque et qui, dans l'exemple décrit est un sériographe 7 déplaçable sur les rails 5 parallèlement au déplacement du tube à rayons X 3. Cette disposition, est remarquable en ce que, pour une position quelconque occupée par la tube à rayons X 3, le sériographe 7 peut se positionner au-dessus de lui, de sorte que la surface utile de film à impressionner soit centrée sur l'axe vertical Z de travail ; ce positionnement du sériographe 7 est effectué soit automatiquement par un asservissement de sa position sur celle du tube à rayons X 3, soit manuellement à l'aide de la poignée 8, la précision nécessaire étant obtenue grâce à des moyens de positionnement quelconques ; ceux-ci pouvant être comme dans l'exemple de la description, constitués d'un montant 33 fixé sur le diaphragme plombé 4, coopérant avec un dispositif mécanique, non représenté sur le dessin, situé sous le sériographe 7 et solidaire de celui-ci. Ce dispositif mécanique comportant par exemple un élément qui, poussé par un ressort, et muni d'un logement destiné à recevoir la partie supérieure du montant 33, est capable d'un mouvement d'effacement sous la poussée du montant 33. Un tel dispositif permet au sériographe 7 d'être manuellement, positionné au-dessus du tube à rayons X 3 ou d'en être écarté ; il permet également pour certains cas d'utilisation de rendre le sériographe 7 solidaire du tube à rayons X 3 dans son déplacement.

Toujours en ce qui concerne le positionnement de sériographe 7, celui-ci comporte sous sa partie inférieure deux capteurs non représentés sur le dessin disposés de manière à être simultanément actionnés quand sa position au-dessus du tube à rayons X 3 est correcte, l'un par le montant 33 et l'autre par le montant 34 fixé également au diaphragme plombé 4 sur le côté opposé à celui du montant 33 et avec le même axe de déplacement ; les signaux électriques correspondant à chacun de ces deux capteurs sont visualisés chacun par un voyant situé sur le support 14, et servent de témoins à la bonne position du sériographe. Ces signaux électriques sont également perçus par les circuits d'automatisme du statif, qui les utilise pour le positionnement automatique du sériographe 7, réalisant ainsi un asservissement de position très simplifié et sur. Quand le fonctionnement exige que l'axe d'émission du tube à rayons X 3 soit libéré par le sériographe 7, l'automatisme maintien celui-ci écarté au plus près, grâce au signal électrique délivré par seulement l'un des deux capteurs actionné par indifféremment le montant 33 ou le montant 34.

Un autre avantage qui découle de la structure du statif, est que la course longitudinale du sériographe 7 lui permet de venir occuper la position destinée aux opérations de chargement de films et cela à l'abri des rayonnements ionisants derrière l'écran transparent 17, généralement constitué de verre au plomb.

Au niveau supérieur du bâti 1, le panneau porte-patient 6 est fixée longitudinalement par ses extrémités 9 et 11, à des supports mobiles 10 et 12 susceptibles d'être déplacés parallèlement à l'axe y-y, lui-même horizontal et perpendiculaire à la longueur du bâti 1 ; les supports mobiles 10 et 12 disposent de moyens de mouvement et de synchronisation classiques qui pour ne pas charger inutilement le dessin, n'y sont pas figurés. Le panneau porte-patient 6 peut ainsi être déplacé par une action manuelle ou motorisée de part et d'autre de sa position centrale de manière à ce que dans l'une ou l'autre position extrêmes de sa course, l'un ou l'autre de ses bords latéraux 13 soit verticalement aligné sur l'axe de déplacement du tube à rayons X 3.

Cet agencement offre l'avantage, en combinant les déplacements longitudinaux du tube à rayons X 3 et du sériographe 7, avec le déplacement latéral du panneau porte-patient 6, d'effectuer l'exploration totale du patient, des pieds à la tête selon deux axes orthogonaux parallèles aux axes x-x et y-y, d'une manière très simplifiée.

Un nouvel intérêt de cet agencement est que le panneau porte-patient 6, disposant d'un point d'appui à chacune de ses extrémités, peut être allégé, et sur toute la longueur du patient, exclusivement constituée en matériau approprié à la nature du rayonnement avec une transparence homogène à celui-ci ; cette caractéristique est surtout importante pour les explorations face/profil du patient, qui dans l'art antérieur sont gênées par la présence sur les bords latéraux de la table d'examen, de pièces métalliques de renfort et de rails servant à son mouvement longitudinal.

La console 16 regroupe les moyens d'affichages et de commandes, nécessaires en fonctionnement ; elle dispose vers l'avant 18 d'un clavier de commande et d'un affichage de différents paramètres qui ne figurent par sur le dessin. Un affichage 19 de certains de ces paramètres est reporté à l'arrière de la console 16 d'où ils sont visibles à travers l'écran 17, par un praticien généralement situé près du patient. Celui-ci dispose également à portée de main, sur le socle mobile 14 d'un clavier 15 qui regroupe certaines des commandes disposées à l'avant de la console 16, et peut ainsi contrôler et procéder aux différentes phases d'un examen. L'emplacement situé à l'avant de la console 16 est généralement occupé par un opérateur abrité des rayonnements ionisants par l'ensemble du statif et par l'écran transparent 17. On y trouve représenté en pointillé et repéré 7A le sériographe 7 en position de chargement, dont le couvercle est relevé, ainsi que le coffret 35 qui renferme les circuits d'électronique 36.

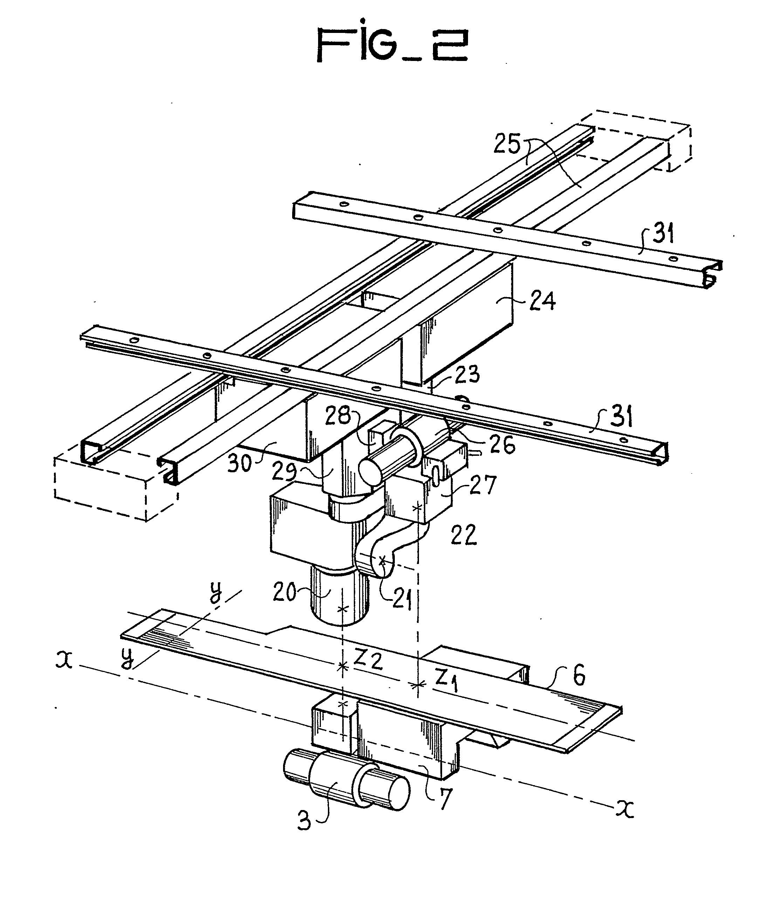

La figure 2 représente une vue du statif selon l'invention où seuls les éléments essentiels sont représentés ainsi que les moyens extérieurs avec lesquels ils coopèrent en fonctionnement. Cette représentation est un exemple non limitatif de la combinaison des moyens intérieurs et extérieurs.

Dans le bas du dessin, sous le panneau porte-patient 6, le sériographe 7, et le tube à rayons X 3 muni d'un diaphragme 4 sont capables d'un déplacement longitudinal parallèle à l'axe x-x. Au-dessus se trouve un premier moyen extérieur qui peut être une source de rayonnement si le statif suivant l'invention n'en comporte pas ou un détecteur de rayonnement dans le cas contraire ; dans ce cas, la présence du tube à rayons X 3 implique que le moyen extérieur est un détecteur de rayonnement et dans l'exemple décrit il s'agit d'un amplificateur de luminance 20. Celui-ci est associé en fonctionnement au tube à rayons X 3 dont il asservit la position sur la sienne par un moyen connu, mécanique ou électronique ; ceci constitue un premier couple source - détecteur définissant un premier axe de travail Z. L'amplificateur de luminance 20, par l'intermédiaire de la fixation articulée 21 et de la bride 22 est porté par une colonne télescopique 23 partiellement visible sur le dessin ; cette colonne télescopique 23 est solidaire d'un chariot 24 capable d'un déplacement le long du chemin de roulement constitué des deux rails 25 parallèles à l'axe y-y.

Egalement au-dessus du panneau porte-patient 6, un deuxième moyen extérieur constitué par une source de rayonnement qui dans l'exemple décrit est un tube à rayons X 26 muni d'un diaphragme plombé 27 fontionne associé au sériographe 7, à l'aide d'un asservissement de position par un moyen connu ; ceci constitue un deuxième couple source - détecteur. Selon une caractéristique importante de l'invention c'est le sériographe 7 qui asservit la position du tube à rayons X 26 sur la sienne. Ce tube à rayons X 26 est porté par l'intermédiaire d'une fixation articulée (non représentée sur le dessin) et de la bride 28 par la colonne télescopique 29, elle-même solidaire du chariot 30. Celui-ci peut se déplacer le long des rails 25 sur le même axe que le chariot 24.

Le chemin de roulement réalisé par les rails 31 permet à l'ensemble constitué par les rails 25 et les éléments suspendus d'effectuer un déplacement manuel ou motorisé au-dessus du panneau porte-patient 6 sur toute la longueur de celui-ci sur un axe parallèle à l'axe x-x grâce à des moyens de déplacement connus (non représentés).

Grâce à l'agencement déjà décrit des moyens intérieurs au statif, le deuxième couple source - détecteur peut se substituer au

8

premier couple source – détecteur.

Le fonctionnement du statif suivant l'invention constitué par la combinaison des moyens décrits précédemment est le suivant : dans cette configuration non limitative, pour la radioscopie, c'est le premier couple source – détecteur qui fonctionne et le praticien peut en combinant le déplacement latéral du panneau porte-patient 6, avec le déplacement longitudinal de l'amplificateur de lumière 20, placer la zone à examiner de l'anatomie du patient en position d'examen ; l'axe de travail Z est alors défini.

Pour la radiographie, c'est le deuxième couple source –détecteur qui entre en action.

Le tube à rayons X 3 dont l'émission est coupée, reste en place après la radioscopie, et suivant une nouvelle caractéristique de l'invention, le praticien par la commande appropriée provoque le déplacement sous la zone à radiographier, du sériographe 7 dont la position est alors asservie à celle du tube à rayons X 3 ; son déplacement provoque également celui des rails 25 et éléments suspendus le long des rails 31, de sorte que le tube à rayons X 26 dont la position est asservie à celle du sériographe 7, prend la place de l'amplificateur de luminance 20. L'axe de travail Z initial est conservé après la substitution du premier couple source – détecteur par le deuxième couple source – détecteur.

REVENDICATIONS

1. Statif de radiologie comportant notamment : une table d'examen (6) ; un premier moyen intérieur au statif situé sous cette table (6), ce premier moyen intérieur étant, soit une source (3), soit un détecteur de rayonnement (20), lié à un premier moyen extérieur au statif situé au dessus de la table (6) et étant selon la nature du premier moyen intérieur soit un détecteur (20) soit une source (3) de rayonnement formant ainsi dans chaque cas, un premier couple, source-détecteur (3-20), déplaçable longitudinalement le long de la table (6), caractérisé en ce qu'il comporte en outre un second moyen intérieur au statif situé sous la table (6) et un second moyen extérieur au statif situé au dessus de la table (6), ces deux derniers moyens étant liés l'un à l'autre et constituant un second couple source-détecteur (26-7) déplaçable tout le long de la table (6), les chemins de déplacement (32-5) de ces premiers et second moyens intérieurs étant parallèles et agencés de telle sorte que le déplacement de ces deux moyens ne se gênent pas mutuellement et permettant la substition d'un couple par l'autre.

2. Statif de radiologie selon la revendication 1, caractérisé en ce que le second moyen intérieur (7) asservit la position du second moyen extérieur (26) sur la sienne.

3. Statif de radiologie selon l'une des revendications 1 et 2, caractérisé en ce que le chemin de déplacement (5) du second moyen intérieur (7) est situé au dessus du chemin de déplacement (32) du premier moyen intérieur (3) de telle sorte que le second moyen intérieur (7) est capable d'être superposé au premier moyen intérieur (3) quelle que soit la localisation de ce dernier.

4. Statif de radiologie selon l'une des revendications précédentes, caractérisé en ce que le premier moyen intérieur (3) asservit la position du second moyen intérieur (7) sur la sienne.

5. Statif de radiologie selon l'une des revendications précédentes, caractérisé en ce que les premier et second moyens intérieurs (3-7) comportent des moyens (33-34) de positionnement assurant le contrôle de la position du second moyen intérieur (7) par rapport à celle du premier moyen intérieur (3).

6. Statif de radiologie selon la revendication 5, caractérisé en ce que ces moyens (33-34) de positionnement sont manuels.

7. Statif de radiologie selon la revendication 5, caractérisé en ce que ces moyens (33-34) de positionnement sont automatiques.

8. Statif de radiologie selon l'une des revendications précédentes, caractérisé en ce que le second moyen intérieur (7),quand il est superposé au premier moyen intérieur (3) est capable de se déplacer en même temps que ce dernier sans variation de leur position relative.

9. Statif de radiologie selon l'une des revendications précédentes, caractérisé en ce que l'agencement du chemin de déplacement (32) du premier moyen intérieur (3) lui permet une course sous toute la longueur du panneau porte-patient (6), sans bouger le patient.

10. Statif de radiologie selon l'une des revendications précédentes, caractérisé en ce que l'agencement du chemin de déplacement (5) du second moyen intérieur (7) lui permet une course sous toute la longueur du panneau porte-patient (6), sans bouger le patient.

11. Statif de radiologie selon l'une des revendications précédentes, caractérisé en ce que l'agencement du chemin de déplacement (5) du second moyen intérieur (7) lui permet d'occuper une position de chargement à l'abri des rayonnements ionisants.

12. Statif de radiologie selon l'une des revendications précédentes, caractérisé en ce que le panneau porte-patient (6) est équipé de moyens (10-12) assurant son déplacement latéral parallèlement à l'axe y-y.

13. Statif de radiologie selon l'une des revendications précécentes, caractérisé en ce que le déplacement du premier ou du second moyen intérieur (3 ou 7) combiné avec le déplacement du panneau porte-patient (6), assure l'exploration totale du patient des pieds à la tête selon deux axes orthogonaux parallèles aux axes x-x et y-y.

14. Statif de radiologie selon l'une des revendications précédentes, caractérisé en ce que le panneau porte-patient (6) est sur toute la longueur du patient exclusivement constitué en matériau présentant une transparence homogène aux rayonnements.

15. Statif de radiologie selon l'une des revendications précédentes, caractérisé en ce qu'il comporte en outre sur le pourtour du bâti (1) un blindage (2) de protection contre les rayonnements ionisants.

FIG_1

FIG_2

Office européen des brevets

RAPPORT DE RECHERCHE EUROPEENNE

**0061390**

Numéro de la demande

EP 82 40 0457

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| A | ELECTROMEDICA, vol. 45, no. 3/4, 1977 ERLANGEN (DE) G. FREDZELL et al.: "Koordinat Angio – eine Basiseinheit für den Spezialuntersuchungsraum" pages 133-136 | | A 61 B 6/00 A 61 B 6/02 |
| | * page 133, le résumé; page 134, figure 1; page 135, figures 3a, 3b et 5; page 136, paragraphe "Der normale Arbeitsplatz für die Routineangiographie" * | 1,14, 15 | |
| | --- | | |
| X | ELECTROMEDICA, vol. 45, no. 3/4, 1977 ERLANGEN (DE) G. GILLY et al.: "Belichtungsauto- matisierte standardisierte Allge- mein- und Zerebralangiographie mit AOT-Blattfilmwechslern und Angio- matic-Steuerung" pages 113-117 | | |
| | * pages 113-117 * | 1,15 | A 61 B 6/00 6/02 |
| | --- | | |
| A | FR - A - 2 239 975 (SIEMENS AG) | | |
| | * page 4, lignes 21-34; figure 1 * | 1,5,8- 10 | |
| | --- | | |
| A | FR - A - 2 374 885 (SIEMENS AG) | | |
| | * page 2, ligne 20 - page 4, ligne 9; page 6, lignes 7-23; page 7, ligne 35 - page 8, ligne 38; | 1,2,7, 13 ./. | |

| | | DOMAINES TECHNIQUES RECHERCHES (Int. Cl. ³) |
|---|---|---|

Le présent rapport de recherche a été établi pour toutes les revendications

| Lieu de la recherche | Date d'achèvement de la recherche | Examinateur |
|---|---|---|
| La Haye | 28-06-1982 | RIEB |

Office européen
des brevets

**RAPPORT DE RECHERCHE EUROPEENNE**

**0061390**
Numéro de la demande

EP 82 40 0457
-2-

| Catégorie | Citation du document avec indication, en cas de besoin, des parties pertinentes | Revendication concernée |
|---|---|---|
| | DOCUMENTS CONSIDERES COMME PERTINENTS | |
| | figures 1,4 * | |
| | --- | |
| A | FR - A - 2 442 042 (LABORATOIRES D'ELECTRONIQUE ET DE PHYSIQUE APPLIQUEE) | |
| | * page 6, lignes 6-15; page 6, lignes 34-37; page 10, ligne 22 - page 11, ligne 20; page 13, ligne 29 - page 14, ligne 30; figures 1,8-10 * | 1,5,7-10 |
| | --- | |
| A | FR - A - 2 163 065 (CFC PRODUCTS INC.) | |
| | * page 6, ligne 22 - page 7, ligne 17; page 10, ligne 27 - page 11, ligne 13; figures 1,6 * | 1,2,5, 7-10 |
| | --- | |
| D,A | FR - A - 2 342 704 (SIEMENS A.G.) | |
| | * page 2, ligne 35 - page 3, ligne 5; page 4, lignes 7-21; figures 1,2 * | 3 |
| | --------- | |

CLASSEMENT DE LA DEMANDE (Int. Cl.³)

DOMAINES TECHNIQUES RECHERCHES (Int. Cl.³)